# EUROPÄISCHE PATENTSCHRIFT

(11) **EP 2 502 636 B1**
(45) Veröffentlichungstag und Bekanntmachung des Hinweises auf die Patenterteilung: **18.01.2017**
(21) Anmeldenummer: 12001789.2
(22) Anmeldetag: 16.03.2012
(51) Int. Cl.: A61L 2/18

(54) **Geteilter Behälter und Verfahren zum Behandeln von kleineren Gegenständen**
Split container and method for treating smaller containers
Récipient divisé et procédé de traitement de petits objets

(30) Priorität: 24.03.2011 DE 102011015044
(43) Veröffentlichungstag der Anmeldung: 26.09.2012
(73) Patentinhaber: Atec Pharmatechnik Gmbh, 24966 Sörup (DE)
(72) Erfinder: Mumm, Hans-Werner, 24966 Sörup (DE)
(74) Vertreter: Thomas, Götz

(56) Entgegenhaltungen:
- WO-A1-00/61199
- WO-A1-2010/040383
- DE-A1- 4 409 659
- DE-U1- 9 421 818

## Beschreibung

Die Erfindung betrifft einen Behälter sowie ein Verfahren zur Behandlung von kleineren Gegenständen gemäß dem Oberbegriff der Ansprüche 1 bzw. 13. Der Behälter und das Verfahren sind vor allem zur Vorreinigung bzw. zum Waschen von Verschlüssen oder Verschlusskappen aus Gummi, Kunststoff oder Aluminium zum Verschließen von Behältern für pharmazeutische Zwecke oder Spritzen bestimmt, die später einer weiteren Reinigung oder Sterilisation unterzogen werden sollen.

Behälter der eingangs genannten Art sind zum Beispiel aus der EP 1 449 543 B1 oder aus der EP 1 510 227 A1 des Anmelders oder aus der WO 00/61199, der DE 299 23 723 U oder der DE 44 09 659 A1 bekannt. Diese Behälter sind vor allem zur Sterilisation von kleineren Gegenständen bestimmt, die nach ihrer Sterilisation nur innerhalb der hermetisch geschlossenen Behälter durch eine unsterile Umgebung transportiert werden können, um eine Kontamination der sterilen Gegenstände zu vermeiden.

Ein etwas anders geformter, jedoch demselben Verwendungszweck dienender Behandlungsbehälter ist aus der WO2010/040383 A1 bekannt.

Allerdings gibt es auch die eingangs bereits genannten Fälle, wo die zu reinigenden kleineren Gegenstände zuerst nur einer Vorreinigung unterzogen und erst nach einer Weiterbearbeitung sterilisiert und erneut gereinigt werden sollen. Einer dieser Fälle betrifft zum Beispiel Verschlusskappen für Spritzen oder Tablettenröhrchen, die durch Tiefziehen aus Aluminium hergestellt werden und nach dem Tiefziehen durch Schmieröl verunreinigt sind, das vor einer Weiterverarbeitung der Verschlusskappen durch Waschen entfernt werden soll. Dazu sollen vorzugsweise dieselben Vorrichtungen wie für die spätere Reinigung und Sterilisation der Verschlusskappen Verwendung finden, deren Konstruktion und Funktionsweise insbesondere in der genannten EP 1 449 543 B1 beschrieben ist.

Da die Vorreinigung keine Sterilisation umfasst, brauchen die gereinigten Verschlüsse oder Gegenstände nicht innerhalb eines geschlossenen Behälters transportiert werden, sondern können sofort entladen und erneut beladen werden. Da die eingangs genannten bekannten Behälter jedoch für eine sterile Entladung bestimmt sind und nur eine relativ kleine, mit einem der Anschlüsse zusammenfallende Befüll- und Entleeröffnung aufweisen, dauert dieser Vorgang verhältnismäßig lange und macht es zudem erforderlich, dass die zu reinigenden Gegenstände als Schüttgut vorliegen, das durch die Befüll- und Entleeröffnung in den Behälter gefüllt und wieder aus diesem ausgetragen werden kann.

Jedoch werden vor allem Verschlüsse oder andere kleinere Gegenstände, die einer Vorreinigung unterzogen werden sollen, nicht selten in Säcken oder Beuteln angeliefert, die für das Behandlungsmedium durchlässig sind, so dass eine Vorreinigung der Gegenstände als Sackware, d.h. ohne eine vorherige Entnahme der Gegenstände aus den Säcken oder Beuteln, grundsätzlich möglich und wegen der einfacheren und schnelleren Handhabung auch von Vorteil wäre, was mit den bekannten Behältern jedoch nicht möglich ist.

Ausgehend hiervon liegt der Erfindung die Aufgabe zugrunde, einen Behälter und ein Verfahren der eingangs genannten Art dahingehend zu verbessern, dass das Beladen und Entladen des Behälters beschleunigt und vorzugsweise auch Sackware behandelt werden kann.

Diese Aufgabe wird erfindungsgemäß im Hinblick auf den Behälter dadurch gelöst, dass der Mittelteil als separates Bauteil in einen Zwischenraum zwischen den beiden Endteilen eingesetzt ist und sich bei Bedarf aus dem Zwischenraum entnehmen lässt.

Auf diese Weise kann zum Beladen und zum Entladen des Behälters der Mittelteil aus dem Zwischenraum zwischen den zwei Endteilen herausgenommen und dadurch in eine Position gebracht werden, in der an seiner Oberseite der volle Behälterquerschnitt zum Beladen und Entladen zur Verfügung steht. Dies ermöglicht vor allem im Fall von Sackware ein sehr schnelles und einfaches Beladen und Entladen des Behälters, da die Sackware lediglich von oben in den Mittelteil gelegt bzw. nach der Behandlung wieder aus dem Mittelteil herausgenommen werden braucht.

Außerdem kann der Mittelteil zur Kapazitätserhöhung mit einem möglichst großen und konstanten Querschnitt versehen werden, während sich die beiden Endteile zweckmäßig zu dem jeweiligen Medienanschluss hin verjüngen.

Wenn die zu behandelnden Gegenstände als Schüttgut vorliegen, kann der Mittelteil ebenfalls sehr einfach beladen werden, indem er unter eine Rutsche oder Schütte bewegt und aus dieser das Schüttgut von oben her in den Mittelteil eingebracht wird. Zum Entladen kann der Mittelteil mit einer Hubvorrichtung angehoben und umgedreht werden, wie beispielsweise der in der EP 1 769 889 B1 des Anmelders beschriebenen Hubvorrichtung. Als Alternative können allerdings auch für das Behandlungsmedium durchlässige Körbe in den Mittelteil eingesetzt werden, die sich zum Entladen der Gegenstände nach oben aus dem Mittelteil heraus nehmen lassen, so dass dieser nicht angehoben und umgedreht werden braucht.

Eine bevorzugte Ausgestaltung der Erfindung sieht vor, dass der Mittelteil des Behälters anders als bei den bekannten Behältern in einzelne Abteile unterteilt ist, die bei einer Behandlung von Sackware jeweils einen oder mehrere Säcke bzw. bei einer Behandlung von Schüttgut jeweils einen Teil des Schüttguts aufnehmen. Diese Unterteilung ist besonders dort von Vorteil, wo der Behälter während der Behandlung gedreht oder geschwenkt wird, um die zu reinigenden Gegenstände durch die Bewegung des Behälters umzulagern und so für eine bessere allseitige Anströmung der Gegenstände mit dem Behandlungsmedium zu sorgen. Die Abteile bieten einen gewissen Freiraum für die Sackware bzw. das Schüttgut, so dass sich die Säcke oder Beutel bzw. die Gegenstände zwar bewegen können, jedoch bei schräg geneigtem Behälter ein Verrutschen der gesamten Sackware oder sämtlicher Gegenstände innerhalb des Behälters zur jeweils unten liegenden Seite hin verhindert wird. Dies würde dort zu einer Erhöhung des Strömungswiderstandes und an der oben liegenden Seite zu einer Verringerung des Strömungswiderstands führen, mit der unerwünschten Folge, dass ein großer Teil des durch den Mittelteil strömenden Behandlungsmediums nicht zwischen den Gegenständen hindurch, sondern an diesen vorbei strömen würde.

Vorteilhaft ist der in den Zwischenraum zwischen den beiden Endteilen eingesetzte Mittelteil gas- und flüssigkeitsdicht mit beiden Endteilen verbindbar, um während der Reinigung von kleineren Gegenständen ein Entweichen von Behandlungsmedium an den Grenzflächen zwischen den beiden Endteilen und dem Mittelteil zu verhindern.

Eine bevorzugte Ausgestaltung der Erfindung sieht vor, dass die Abmessungen des Zwischenraums zwischen den beiden Endteilen bei der Entnahme und beim Einführen des Mittelteils unverändert bleiben, so dass nur der Mittelteil zwischen den Endteilen heraus bzw. zwischen die Endteile hinein bewegt werden muss, während die letzteren stationär in ihrer Position verbleiben.

Um die Entnahme des Mittelteils aus dem Zwischenraum bzw. das Einführen des Mittelteils in den Zwischenraum zu ermöglichen, ohne dass der Mittelteil zuvor in eine definierte Stellung gebracht werden muss, ist der Mittelteil gemäß einer weiteren vorteilhaften Ausgestaltung der Erfindung entlang von zwei parallelen Verbindungsebenen mit beiden Endteilen verbindbar und lässt sich in einer zu diesen Ebenen parallelen Richtung zwischen die beiden Endteile einführen. Dazu trägt auch bei, dass der Mittelteil vorzugsweise zylindrisch ist, während sich die beiden Endteile zweckmäßig zu den Medienanschlüssen hin verjüngen.

Um trotz der stationären Endteile während der Reinigung von kleineren Gegenständen zwischen den beiden Endteilen und dem Mittelteil für eine gas- und flüssigkeitsdichte Abdichtung zu sorgen, ist zwischen den Endteilen und dem Mittelteil bevorzugt jeweils eine Dichtungsanordnung mit einer ringförmigen Dichtung vorgesehen. Die Dichtung ist vorzugsweise in axialer Richtung des Behälters beweglich, um einen schmalen Ringspalt zwischen jedem der Endteile und dem Mittelteil zu überbrücken, der zum Einführen und zur Entnahme des Mittelteils notwendig ist. Die Dichtung kann zweckmäßig nach dem Einsetzen des Mittelteils gegen eine gegenüberliegende Dichtfläche angepresst werden, um entlang von jedem Ringspalt für die gas- und flüssigkeitsdichte Abdichtung zwischen den beiden Endteilen und dem Mittelteil zu sorgen. Dies erfolgt bevorzugt dadurch, dass eine die Dichtung aufnehmende Nut zwischen ihrem Nutgrund und der Dichtung mit einem unter Druck stehenden Fluid, zweckmäßig mit Druckluft, beaufschlagt wird. Durch Anlegen eines Unterdrucks an der Nut bzw. zwischen dem Nutgrund und der Dichtung kann die Dichtung vor jeder Entnahme des Mittelteils von der Dichtfläche weg zurückgezogen werden.

Um die Zufuhr des Fluids in die Nut bzw. das Anlegen des Unterdrucks an der Nut zu erleichtern, sind die Nuten mit der Dichtung vorteilhaft in den stationären Endteilen und nicht im beweglichen Mittelteil angeordnet. Eine weitere zweckmäßige Ausgestaltung der Erfindung sieht vor, dass die Nuten jeweils an der dem Zwischenraum zugewandten Stirnfläche jedes Endteils angeordnet sind und sich entlang des Ringspalts bzw. des äußeren Umfangs des Behälters erstrecken.

Vorteilhaft sind die Endteile im Bereich der Verbindungsebenen jeweils mit einem axial überstehenden Kragen versehen, der beim Einführen des Mittelteils zur Zentrierung desselben zwischen den Endteilen dient. Der Kragen ist bevorzugt so ausgebildet, dass er einen radialen Flanschrand des Mittelteils umgreift, wenn sich der Mittelteil im Zwischenraum befindet. Damit verhindert der Kragen, dass sich beim Abdichten der Ringspalte mittels der Dichtung die Endteile in axialer Richtung vom Mittelteil weg bewegen können.

Während der Behandlung der Gegenstände werden der Mittelteil und die beiden Endteile vom Behandlungsmedium durchströmt, in der Regel von unten nach oben, um die Gegenstände im Strom des Behandlungsmediums zu suspendieren, so dass sie sich innerhalb des Behandlungsmediums frei bewegen können und allseitig vom Behandlungsmedium angeströmt werden. Um zu verhindern, dass einzelne Gegenstände aber auch Sackware durch den aufwärts gerichteten Strom des Behandlungsmediums in den während der Reinigung der Gegenstände oberhalb des Mittelteils angeordneten Endteil mitgerissen werden, ist dieser Endteil an seiner Unterseite zweckmäßig mit einem für das Behandlungsmedium durchlässigen Sieb oder Lochblech versehen. Das sieb oder Lochblech ist bevorzugt unmittelbar über der Oberseite des Mittelteils angeordnet und hält die zu behandelnden Gegenstände bzw. die Sackware im Mittelteil zurück.

Zweckmäßig ist der Mittelteil an seiner Unterseite ebenfalls mit einem Sieb oder Lochblech versehen. Dieses Sieb oder Lochblech verhindert, dass die zu behandelnden Gegenstände bzw. die Sackware nach unten in den anderen Endteil fallen kann, der während der Behandlung unterhalb des Mittelteils angeordnet ist. Vorzugsweise ist das Sieb oder Lochblech unmittelbar über der Unterseite des Mittelteils angeordnet, so dass im Mittelteil möglichst viel Raum für die Unterbringung der zu behandelnden Gegenstände oder Sackware bleibt.

Im Hinblick auf das Verfahren wird die Aufgabe erfindungsgemäß dadurch gelöst, dass der als separates Bauteil in einen Zwischenraum zwischen den beiden Endteilen eingesetzte Mittelteil zum Beladen und Entladen des Behälters aus dem Zwischenraum herausgenommen wird.

Wie bereits angesprochen wurde, ist dies vor allem dann von Vorteil, wenn die kleineren Gegenstände in Säcken oder Beuteln enthalten sind, die gemäß einer vorteilhaften Ausgestaltung der Erfindung zum Beladen und zum Entladen des Behälters von oben her in den Mittelteil gelegt bzw. nach oben aus dem Mittelteil entnommen werden, nachdem dieser zwischen den beiden Endteilen herausgenommen worden ist.

Um einen Austritt von Behandlungsmedium bei der Entnahme des Mittelteils zu vermeiden, wird das Behandlungsmedium zweckmäßig vorher aus dem Behälter abgelassen, wobei die im Mittelteil befindlichen Gegenstände oder Sackware von dem Sieb oder Lochblech an der Unterseite des Mittelteils zurückgehalten werden.

Im Folgenden wird die Erfindung anhand eines in der Zeichnung dargestellten Ausführungsbeispiels näher erläutert. Es zeigen:
Fig. 1a, 1b und 1c: Seitenansichten eines erfindungsgemäßen Behälters mit zwei Endteilen und einem Mittelteil vor, während und nach der Entnahme des Mittelteils;
Fig. 2a und 2b: Vorderseitenansichten des Behälters vor und nach der Entnahme des Mittelteils;
Fig. 3: eine Längsschnittansicht des Behälters;
Fig. 4: eine Oberseitenansicht des Mittelteils entlang der Linie IV-IV der Fig. 1c;
Fig. 5: eine Oberseitenansicht des unteren Endteils entlang der Linie V-V der Fig. 1c;
Fig. 6: eine vergrößerte perspektivische Ansicht eines Teils eines Transportwagens zur Entnahme und zum Transport des Mittelteils;
Fig. 7: eine vergrößerte Querschnittansicht des Ausschnitts VII in Fig. 3.

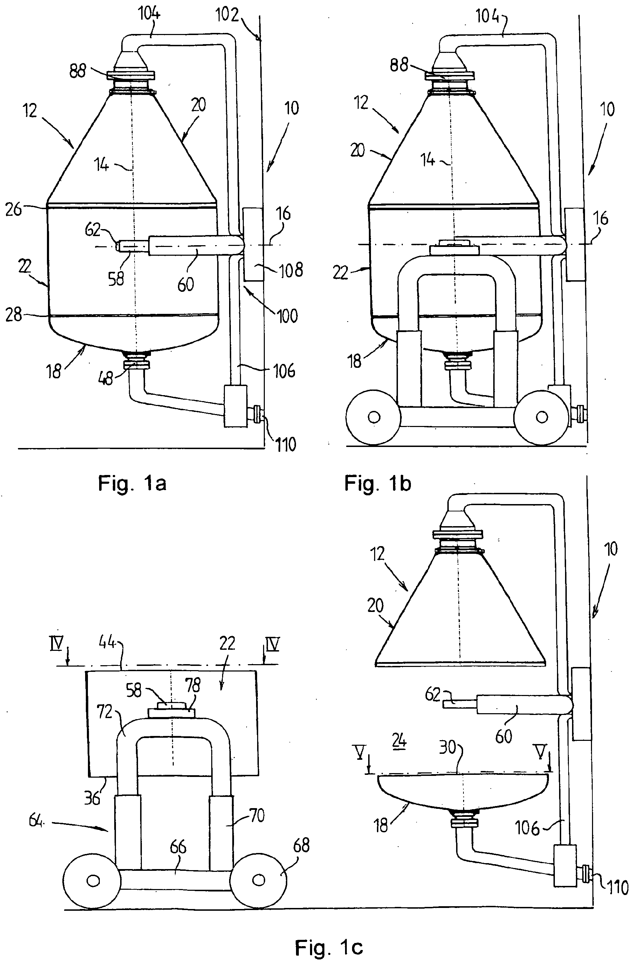

Die in den Figuren 1a, 1b und 1c dargestellte Vorrichtung 10 dient unter anderem zur Vorreinigung von Verschlüssen für pharmazeutische Verpackungsbehälter, die in Form von Sackware angeliefert werden, wobei in einem Sack oder Beutel jeweils mehrere Hundert Verschlüsse enthalten sind. Die Säcke bestehen aus einem für Gase und Flüssigkeiten durchlässigen Gewebe, so dass die Verschlüsse zur Vorreinigung nicht aus den Säcken oder Beuteln entnommen werden müssen, sondern innerhalb der Säcke durch Waschen gereinigt werden.

Das Waschen der Verschlüsse erfolgt im Inneren eines mit der Vorrichtung gekoppelten Behälters 12, der in der Vorrichtung 10 mit Reinigungsmedien, wie Heißwasser, Kaltwasser, einem Wasser-Luft-Gemisch, Trocknungsluft oder Dampf beaufschlagt und dabei um eine zu seiner Längsmittelachse 14 senkrechte Drehachse 16 geschwenkt werden kann, wie in Fig. 1a und 1b dargestellt.

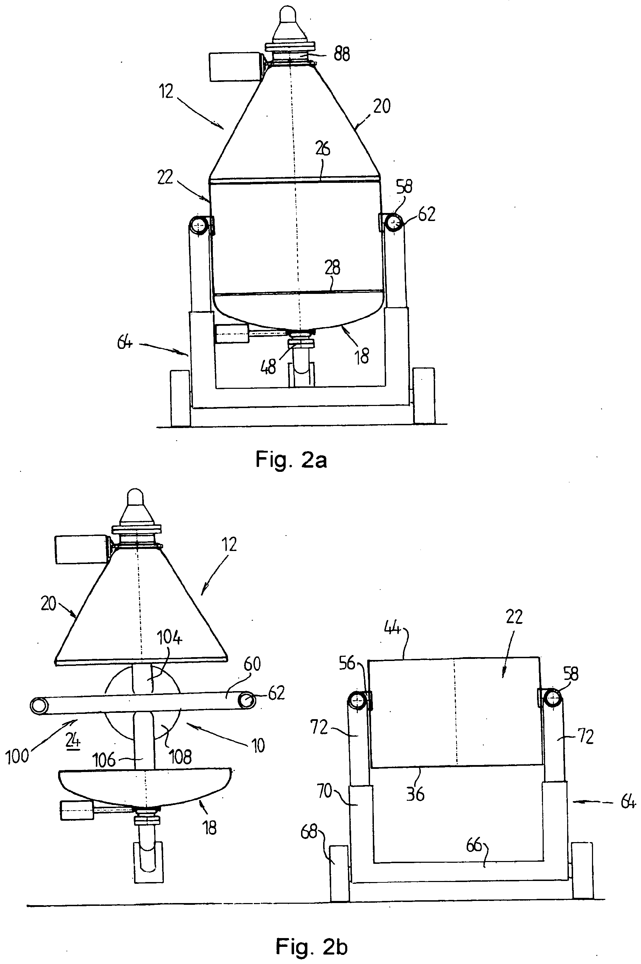

Wie am besten in den Figuren 1 bis 3 dargestellt, weist der Behälter 12 bei Betrachtung in einer aufrechten Stellung einen wannenförmigen unteren Endteil 18, einen nach oben verjüngten oberen Endteil 20 und einen vom unteren und vom oberen Endteil 18, 20 getrennten Mittelteil 22 auf. Der Mittelteil bildet ein separates Bauteil, das in einen Zwischenraum 24 zwischen dem an der Vorrichtung 10 angekoppelten oberen und dem unteren Endteil eingesetzt bzw. aus dem Zwischenraum 24 entnommen werden kann, wie am besten in den Figuren 1a, 1b und 2a bzw. 1c und 2c dargestellt.

Der Zwischenraum 24 wird nach oben und nach unten zu von einer unteren bzw. oberen Verbindungsebene 26, 28 begrenzt, die parallel zueinander und senkrecht zur Längsmittelachse 14 des Behälters 12 ausgerichtet sind.

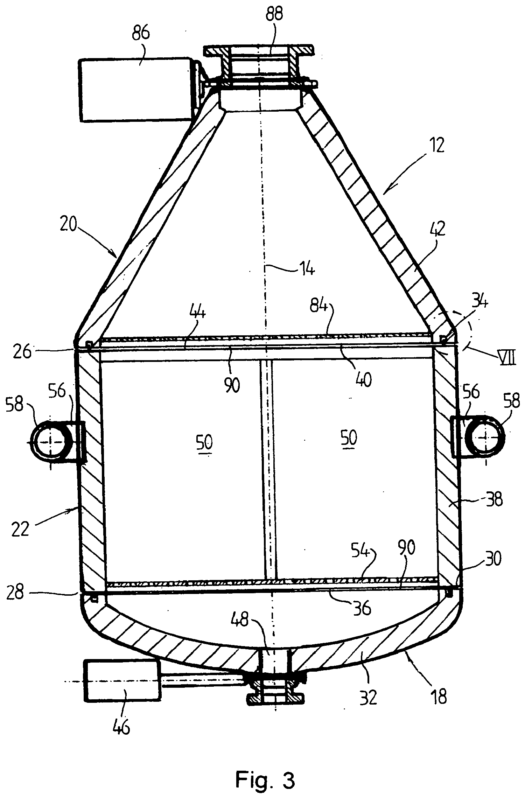

wie am besten in Fig. 3 dargestellt, ist der ebene obere Rand 30 einer konvex nach unten gewölbten Begrenzungswand 32 des unteren Endteils 18 in der unteren Verbindungsebene 28 durch eine Dichtungsanordnung 34 gas- und flüssigkeitsdicht mit einer ebenen ringförmigen Dichtfläche 36 am gegenüberliegenden unteren Stirnende einer zylindrischen Umfangswand 38 des Mittelteils 22 verbindbar, während der ebene untere Rand 40 einer nach oben zu konisch verjüngten Begrenzungswand 42 des oberen Endteils 20 in der oberen Verbindungsebene 26 durch eine weitere Dichtungsanordnung 34 gas- und flüssigkeitsdicht mit einer ebenen ringförmigen Dichtfläche 44 am oberen Stirnende der Umfangswand 38 des Mittelteils 22 verbindbar ist, wie später ausführlicher beschrieben wird.

Der untere Endteil 18 weist an seiner tiefsten Stelle einen Medienanschluss 48 auf, durch den die Behandlungsmedien in den Behälter 12 zugeführt oder aus diesem abgeführt werden können. Bei dem dargestellten Behälter 12 ist der Medienanschluss 48 durch ein Klappenventil 46 verschließbar, jedoch kann auf das Klappenventil 46 verzichtet werden, wenn die Vorrichtung 10 ein derartiges Ventil oder Absperrorgan zur Steuerung des Stroms der Behandlungsmedien umfasst.

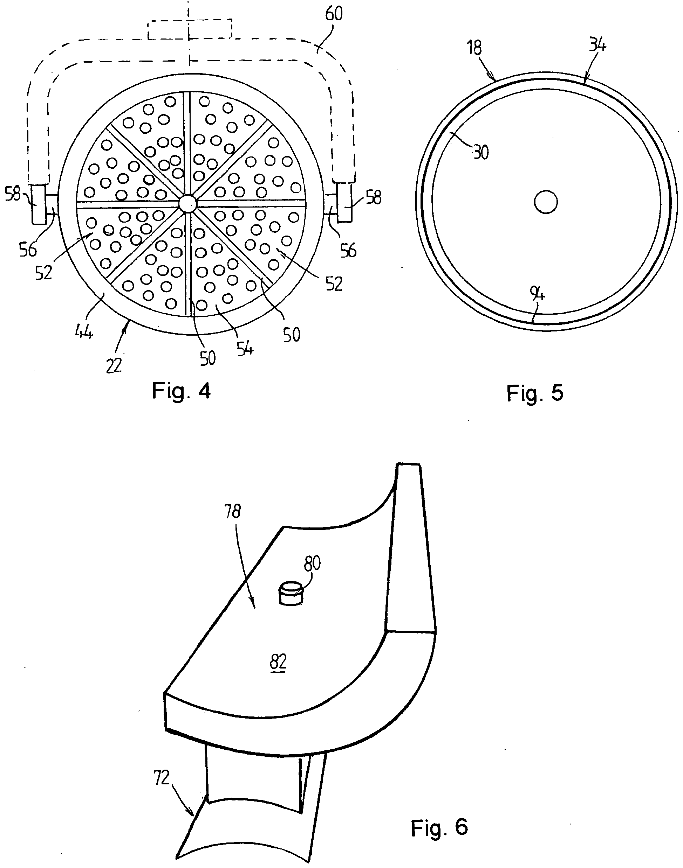

Wie am besten in Fig. 4 dargestellt, ist der nach oben offene zylindrische Mittelteil 22 im Inneren durch sternförmige Zwischenwände 50 in mehrere Abteile 52 unterteilt, die jeweils zur Aufnahme von einem oder mehreren Säcken oder Beuteln mit Verschlüssen oder von Verschlüssen in Form von losem Schüttgut dienen. An der Unterseite ist in der Mittelteil 22 ein zur Längsmittelachse 14 des Behälters 12 senkrechtes Lochblech 54 eingesetzt, das bei der Behandlung einen Hindurchtritt der Behandlungsmedien gestattet und bei aufrechtem Behälter 12 ein Abtropfen der Verschlüsse erlaubt, jedoch ein Herausfallen der Säcke oder Beutel bzw. des Schüttguts in den unteren Endteil 18 verhindert.

Die zylindrische Umfangswand 38 des Mittelteils 22 ist an ihrer Außenseite mit zwei diametral entgegengesetzten seitlichen Auslegern 56 versehen. Beide Ausleger 56 tragen jeweils eine hohlzylindrische Aufnahmebuchse 58, deren Längsachsen parallel zu den Verbindungsebenen 26, 28 ausgerichtet sind. Die Aufnahmebuchsen 58 dienen zur lösbaren Befestigung des Behälters 12 an einem schwenkbaren U-förmigen Tragelement 60 der Vorrichtung 10. Die parallelen Enden des Tragelements 60 sind jeweils mit einem Tragdorn 62 versehen, der sich in eine der Aufnahmebuchsen 58 einführen lässt.

Die Aufnahmebuchsen 58 dienen außerdem zum Abstützen des Mittelteils 22 auf einem Transportwagen 64, der zur Entnahme des Mittelteils 22 aus dem Zwischenraum 24 sowie zum Transport desselben nach der Entnahme dient. Wie am besten in Fig. 1b, 1c, 2a und 2c dargestellt, besitzt der Transportwagen 64 ein Fahrgestell mit vier Rädern 68, einem Grundrahmen 66 und vier vertikalen hohlen Stützen 70, in denen paarweise zwei Tragbügel 72 höhenverstellbar sind. Die Tragbügel 72 weisen jeweils zwei parallele vertikale Schenkel auf, die von oben her in die hohlen Stützen 70 ragen und innerhalb derselben verstellbar sind. Die beiden Schenkel jedes Tragbügels 74 sind durch ein horizontales Joch miteinander verbunden, das eine Tragmulde 78 zur Aufnahme einer Aufnahmebuchse 58 des Mittelteils 22 trägt.

Wie am besten in Fig. 6 dargestellt, weist die Tragmulde 78 einen Vorsprung 80 auf, der nach oben über eine konkave Auflagefläche 82 der Tragmulde 78 ausgefahren und in eine komplementäre Bohrung (nicht sichtbar) an der Unterseite der auf der Auflagefläche 82 aufliegenden Aufnahmebuchse 58 eingeführt werden kann.

Der konische obere Endteil 20 weist ein unmittelbar über der oberen Verbindungsebene 26 lösbar ins Innere des Endteils 20 eingesetztes Lochblech 84 (Fig. 3) auf und ist am oberen Ende ebenfalls mit einem Medienanschluss 88 versehen, der wie der Anschluss 48 des unteren Endteils 18 zur Zufuhr und/oder zur Abfuhr von Behandlungsmedien dient. Der Anschluss 88 weist jedoch einen größeren Öffnungsquerschnitt als der Anschluss 48 auf, so dass er nach dem Abnehmen des Behälters 12 von der Vorrichtung 10 und nach der Entnahme des Lochblechs 84 ggf. auch als Befüll- und Entleeröffnung verwendet kann, durch die bei Bedarf zu reinigende Verschlüsse in Form von Schüttgut in den aufrechten Behälter 12 eingebracht bzw. nach einer Drehung des Behälters 12 um 180 Grad wieder ausgetragen werden können. Bei dem dargestellten Behälter 12 ist der Medienanschluss 88 durch ein Klappenventil 86 verschließbar, jedoch kann auf das Klappenventil 86 verzichtet werden, wenn die Vorrichtung 10 ein derartiges Ventil oder Absperrorgan zur Steuerung des Stroms der Behandlungsmedien umfasst.

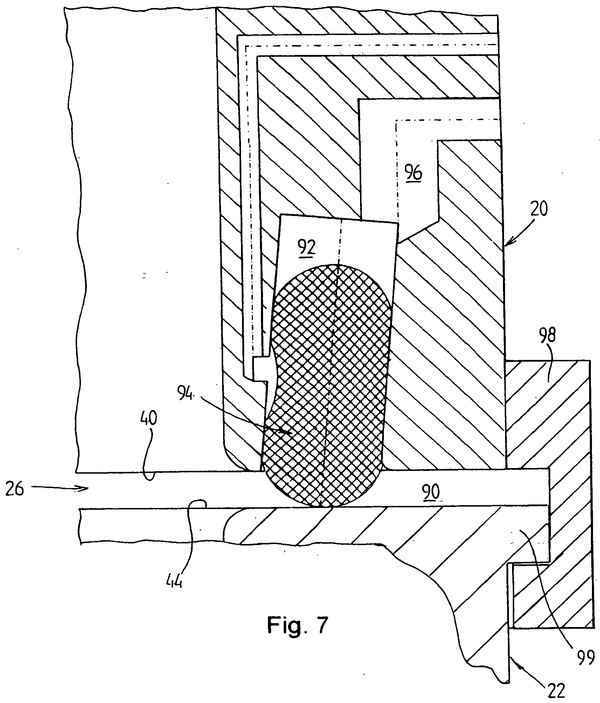

Wie in Fig. 7 am Beispiel der Dichtungsanordnung 34 zwischen dem oberen Endteil 20 und dem Mittelteil 22 dargestellt ist, dient jede Dichtungsanordnung 34 dazu, entlang der beiden Verbindungsebenen 26 und 28 einen Ringspalt 90 zwischen dem Endteil 20 bzw. 18 und dem in den Zwischenraum 24 eingesetzten Mittelteil 22 gas- und flüssigkeitsdicht abzudichten. Die Dichtungsanordnung 34 weist zu diesem Zweck eine in der ebenen Stirnfläche 40 bzw. 30 des Endteils 20 bzw. 18 ausgesparte ringförmige Nut 92 auf, in die ein gummielastischer Dichtring 94 mit dichtender Passung verschiebbar eingesetzt ist. Die Nut 92 ist über einen Kanal 96 wahlweise mit einer Druckluftquelle oder mit einer Unterdruckquelle (nicht dargestellt) verbindbar, um den Dichtring 94 zur Abdichtung des Ringspalts 90 mit Druckluft gegen die gegenüberliegende Dichtfläche 44 bzw. 36 des Mittelteils 22 anzupressen bzw. um ihn zur Entnahme des Mittelteils 22 aus dem Zwischenraum 24 ins Innere der Nut 92 zurückzuziehen und den Ringspalt 90 freizugeben.

Wie ebenfalls in Fig. 7 am Beispiel der Verbindungsebene 26 dargestellt ist, sind die beiden Endteile 18, 20 entlang eines kleineren Teils ihres Umfangs mit einem axial überstehenden Kragen 98 versehen, der beim Einführen des Mittelteils 22 in den Zwischenraum 24 als Zentrieranschlag für den Mittelteil 22 dient. Wenn sich der Mittelteil 22 im Zwischenraum 24 befindet, umgreift der Kragen 98 einen radial überstehenden Rand 99 des Mittelteils 22 und verhindert dadurch, dass sich die Endteile 18, 20 unter Verbreiterung des Ringspalts 90 vom Mittelteil 22 weg bewegen, wenn der Dichtring 94 durch Zufuhr von Druckluft in die Nut 92 gegen die Dichtfläche 44 bzw. 36 angepresst wird.

Wie am besten in Fig. 1 dargestellt, umfasst die Vorrichtung 10 einen in der Zeichnung dargestellten Behälteraufnahmeteil 100, der über eine Wand 102 übersteht und in vertikaler Richtung etwas angehoben bzw. abgesenkt werden kann, sowie einen hinter der Wand 102 angeordneten Maschinenteil (nicht dargestellt), der zur Bereitstellung und Aufbereitung der Behandlungsmedien dient. Der Behälteraufnahmeteil 100 umfasst neben dem um die Drehachse 16 schwenkbaren Tragelement 60 zwei Leitungen 104, 106, die wahlweise zur Zufuhr und Abfuhr der Behandlungsmedien in den im Behälteraufnahmeteil 100 aufgenommenen Behälter 12 verwendet werden können. Die beiden Leitungen 104, 106 erstrecken sich vom oberen bzw. unteren Anschluss 88, 48 bis zu einer drehbaren Trägerplatte 108, wo sie durch die Trägerplatte 108 hindurch zum Maschinenteil der Vorrichtung 10 verlaufen. Die Leitungen 104, 106 sind zusammen mit der Trägerplatte 108 und dem daran befestigten Tragelement 60 um die Drehachse 16 schwenkbar und können innerhalb des Maschinenteils Ventile oder Absperrorgane enthalten, wenn der Behälter nicht mit den Klappenventilen 46 und 86 ausgestattet ist.

Die Vorrichtung 10 umfasst weiter einen Entleerungsanschluss 110, der wahlweise von der Leitung 106 getrennt oder bei aufrechtem Behälter 12 zur Restentleerung desselben mit der Leitung 106 verbunden werden kann. Weitere Informationen über den Aufbau und die Funktionsweise einer solchen Vorrichtung 10 finden sich in der EP 1 449 543 B1 des Anmelders.

Im Folgenden wird die Vorgehensweise bei der Vorreinigung von Verschlüssen beschrieben: Wenn der Behälter 12 ausgehend von der in Fig. 1a und 2a dargestellten Stellung, in welcher der Mittelteil 22 zwischen die beiden Endteile 18, 20 eingesetzt ist, mit den zu waschenden Verschlüssen in Form von Sackware beladen werden soll, wird zuerst ein Unterdruck an den Nuten 92 der beiden Endteile 18, 20 angelegt, um die Dichtringe 94 in die Nuten 92 zurückzuziehen und die Ringspalte 90 vollständig freizugeben. Anschließend wird der Transportwagen 64 unter den Behälter 12 gefahren, nachdem zuvor die Höhe der beiden Tragbügel 74 so eingestellt worden ist, dass sich diese in einem geringen vertikalen Abstand unter den Aufnahmebuchsen 58 befinden. In dieser Stellung werden die Vorsprünge 80 an den Tragmulden 78 (Fig. 6) ausgefahren und von unten her in die darüber liegende Bohrung der Aufnahmebuchsen 58 eingeführt. Dadurch werden die Aufnahmebuchsen 58 von den beiden Tragdornen 62 des Tragelements 60 abgezogen und der Mittelteil 22 aus dem Zwischenraum 24 zwischen den beiden Endteilen 18, 20 heraus bewegt, wenn der Transportwagen 64 aus der Stellung in Fig. 1b in die Stellung in Fig. 1c verfahren wird.

In dieser letzteren Stellung kann die Sackware mit den zu waschenden Verschlüssen von oben her in die Abteile 52 des Mittelteils 22 gelegt oder alternativ die Verschlüsse als Schüttgut in die Abteile 52 gefüllt werden. Zum Einführen zwischen die beiden Endteile 18, 20 wird der Mittelteil 22 anschließend auf dem Transportwagen 64 wieder in die in Fig. 1b und 2a dargestellte Stellung gefahren und durch die Kragen 98 im Zwischenraum 24 zentriert, während die Tragdorne 62 der Tragelemente 60 wieder in den Aufnahmebuchsen 58 eingeführt werden. Wenn der Mittelteil 22 diese Stellung erreicht hat, wird Druckluft in die Nuten 92 der beiden Endteile 18, 20 zugeführt, um die Dichtringe 94 der Dichtungsanordnungen 34 gegen die jeweils gegenüberliegenden Dichtflächen 44, 36 des Mittelteils 22 anzupressen und die Ringspalte 90 in den beiden Verbindungsebenen 26, 28 gas- und flüssigkeitsdicht mit den Endteilen 18, 20 zu verbinden. Danach werden die Vorsprünge 80 an den Tragmulden 78 manuell eingefahren, bis sie nicht mehr in die Bohrungen der Aufnahmebuchsen 58 eingreifen, und dann der Behälteraufnahmeteil 100 mitsamt dem ganzen Behälter 12 leicht angehoben, so dass der Transportwagen 64 ohne den Mittelteil 22 unter dem Behälter 12 herausgezogen werden kann.

Anschließend können die Verschlüsse in der Sackware innerhalb des Behälters 12 gewaschen werden, indem aus dem Maschinenteil der Vorrichtung 10 Behandlungsmedien durch eine der Leitungen 102, 104 in den unteren oder den oberen Endteil 18, 20 des Behälters 12 zugeführt werden, von wo aus sie unter Reinigung der Verschlüsse durch den Mittelteil 22 in den anderen Endteil 20, 18 strömen. Von dort aus werden sie durch die jeweils andere Leitung 104, 102 zur Aufbereitung wieder in den Maschinenteil der Vorrichtung 10 zurück geleitet. Während der Reinigung der Verschlüsse kann der gesamte Behälter 12 um die Drehachse 16 hin und her geschwenkt werden, um die Verschlüsse während der Beaufschlagung mit den Behandlungsmedien in Bewegung zu versetzen und auf diese Weise für eine stärkere Umlagerung der Verschlüsse bzw. der Sackware innerhalb der Abteile 52 zu sorgen. Die Reinigung der Verschlüsse umfasst nicht nur deren Wäsche, sondern auch eine anschließende Trocknung und Kühlung der Verschlüsse.

Nach der Reinigung der Verschlüsse kann der Mittelteil 22 des Behälters 12 zur Entnahme der gereinigten Verschlüsse und ggf. zur Beladung mit weiteren Verschlüssen erneut aus dem Zwischenraum 24 entnommen werden, wie zuvor beschrieben.

## Patentansprüche

1. Behälter (12) zur Behandlung von kleineren Gegenständen, wie Teilen von Spritzen, Verschlüssen oder Verschlusskappen aus Gummi, Kunststoff oder Aluminium zum Verschließen von Behältern für pharmazeutische Zwecke oder dergleichen, wobei der Behälter (12) in einer aufrechten Stellung einen wannenförmigen unteren Endteil (18) und einen nach oben verjüngten oberen Endteil (20) aufweist, wobei der untere Endteil (18) an seiner tiefsten Stelle und der obere Endteil (20) am oberen Ende mit Anschlüssen (48, 88) zur Zufuhr und Abfuhr von mindestens einem Behandlungsmedium versehen sind, und wobei der Behälter (12) einen zwischen den Endteilen angeordneten Mittelteil (22) zur Aufnahme der zu reinigenden Gegenstände aufweist, durch den das in einen der Endteile (18; 20) zugeführte Behandlungsmedium hindurch geleitet werden kann, um es durch den anderen Endteil (20; 18) abzuführen, **dadurch gekennzeichnet, dass** der Mittelteil (22) als separates Bauteil in einen Zwischenraum (24) zwischen den beiden Endteilen (18, 20) eingesetzt ist und sich bei Bedarf aus dem Zwischenraum (24) entnehmen lässt.

2. Behälter nach Anspruch 1, **dadurch gekennzeichnet, dass** der Mittelteil (22) gas- und flüssigkeitsdicht mit beiden Endteilen (18, 20) verbindbar ist.

3. Behälter nach Anspruch 1 oder 2, **dadurch gekennzeichnet, dass** der Mittelteil (22) in zwei parallelen Verbindungsebenen (26, 28) mit beiden Endteilen (18, 20) verbindbar ist und sich in einer zu den Verbindungsebenen (26, 28) parallelen Richtung aus dem Zwischenraum (24) entnehmen lässt.

4. Behälter nach einem der vorangehenden Ansprüche, **dadurch gekennzeichnet, dass** bei der Entnahme und beim Einführen des Mittelteils (22) die beiden Endteile (18, 20) stationär und die Abmessungen des Zwischenraums (24) zwischen den beiden Endteilen (18, 20) unverändert bleiben.

5. Behälter nach einem der vorangehenden Ansprüche, **dadurch gekennzeichnet, dass** zwischen dem Mittelteil (22) und den beiden Endteilen (18, 20) ringförmige Dichtungen (94) vorgesehen sind, die Ringspalte (90) zwischen den benachbarten Teilen (18, 22; 20, 22) überbrücken.

6. Behälter nach Anspruch 5, **dadurch gekennzeichnet, dass** sich die Dichtungen (94) zur Überbrückung des Ringspalts (90) durch Beaufschlagung mit einem unter Druck stehenden Fluid gegen eine gegenüberliegende Dichtfläche (44, 36) anpressen und durch Anlegen eines Unterdrucks von der Dichtfläche (44, 36) weg zurückziehen lassen.

7. Behälter nach Anspruch 5 oder 6, **dadurch gekennzeichnet, dass** die Dichtungen (94) in Nuten (92) der Endteile (18, 20) eingesetzt sind und sich gegen gegenüberliegende Dichtflächen (44, 36) des Mittelteils (22) anpressen lassen.

8. Behälter nach einem der vorangehenden Ansprüche, **dadurch gekennzeichnet, dass** der Mittelteil (22) in der Nähe von mindestens einem der beiden Endteile (18, 20) mit einem für das Behandlungsmedium durchlässigen Sieb oder Lochblech (54) versehen ist, das die Gegenstände im Mittelteil (22) zurückhält.

9. Behälter nach einem der vorangehenden Ansprüche, **dadurch gekennzeichnet, dass** einer der beiden Endteile (20) in der Nähe des Mittelteils (22) mit einem für das Behandlungsmedium durchlässigen Sieb oder Lochblech (54) versehen ist, das die Gegenstände im Mittelteil (22) zurückhält.

10. Behälter nach einem der vorangehenden Ansprüche, **dadurch gekennzeichnet, dass** der Mittelteil (22) in einzelne Abteile (52) unterteilt ist, die jeweils einen Teil der Gegenstände aufnehmen.

11. Behälter nach einem der Ansprüche 3 bis 9, **dadurch gekennzeichnet, dass** die Endteile (18, 20) entlang eines Teils ihres Umfangs mit einem über die Verbindungsebene (26, 28) überstehenden Kragen (98) versehen sind, der zur Zentrierung des Mittelteils (22) beim Einführen in den Zwischenraum (24) dient.

12. Behälter nach Anspruch 11, **dadurch gekennzeichnet, dass** der Kragen (98) einen überstehenden Rand (99) des in den Zwischenraum (24) eingeführten Mittelteils (22) umgreift.

13. Verfahren zum Behandeln von kleineren Gegenständen, wie Teilen von Spritzen, Verschlüssen oder Verschlusskappen aus Gummi, Kunststoff oder Aluminium zum Verschließen von Behältern für pharmazeutische Zwecke oder dergleichen, wobei ein Behälter (12), der in einer aufrechten Stellung einen wannenförmigen unteren Endteil (18), einen nach oben verjüngten oberen Endteil (20) und einen dazwischen angeordneten Mittelteil (22) aufweist, mit den Gegenständen beladen wird, wobei die Gegenstände im Mittelteil (22) des Behälters (12) mit einem Behandlungsmedium beaufschlagt werden, das durch einen Anschluss (88) am oberen Ende des oberen Endteils (18) oder durch einen Anschluss (48) an der tiefsten Stelle des unteren Endteils (20) in einen der Endteile (18; 20) zugeführt, von dort durch den Mittelteil (22) hindurch geleitet und aus dem anderen der Endteile (20; 18 abgeführt wird, **dadurch gekennzeichnet, dass** der als separates Bauteil in einen Zwischenraum (24) zwischen den beiden Endteilen (18, 20) eingesetzte Mittelteil (22) zum Beladen und Entladen des Behälters (12) aus dem Zwischenraum (24) entnommen wird.

14. Verfahren nach Anspruch 13, **dadurch gekennzeichnet, dass** die kleineren Gegenstände in Säcken oder Beuteln enthalten sind, die zum Beladen und zum Entladen des Behälters (12) von oben her in den Mittelteil (22) gelegt bzw. nach oben aus dem Mittelteil (22) entnommen werden, nachdem dieser zwischen den beiden Endteilen (18, 20) entnommen worden ist.

## Claims

1. Container (12) for the treatment of comparatively small items such as parts of syringes, closures, or closure caps of rubber, plastics, or aluminium, for closing containers for pharmaceutical purposes or the like, wherein the container (12) in an upright position has a tub-shaped lower end part (18) and an upwardly tapered upper end part (20), wherein the lower end part (18) at the deepest point thereof, and the upper end part (20) at the upper end, are provided with connectors (48, 88) for the supply and the discharge of at least one treatment medium, and wherein the container (12) has a central part (22), disposed between the end parts, for receiving the items to be cleaned, through which central part (22) the treatment medium which has been supplied to one of the end parts (18; 20) may be directed so that said treatment medium may be discharged through the other end part (20; 18), **characterized in that** the central part (22) is inserted as a separate component into an intermediate space (24) between the two end parts (18, 20) and may be removed on demand from the intermediate space (24).

2. Container according to Claim 1, **characterized in that** the central part (22) is connectable in a gas-tight and fluid-tight manner to both end parts (18, 20).

3. Container according to Claim 1 or 2, **characterized in that** the central part (22) is connectable to the two end parts (18, 20) in two parallel connection planes (26, 28) and may be removed from the intermediate space (24) in a direction which is parallel with the connection planes (26, 28).

4. Container according to one of the preceding claims, **characterized in that** in the case of the central part (22) being removed and being introduced the two end parts (18, 20) remain stationary, and the dimensions of the intermediate space (24) between the two end parts (18, 20) remain unchanged.

5. Container according to one of the preceding claims, **characterized in that** annular seals (94) which bridge annular gaps (90) between the adjacent parts (18, 22; 20, 22) are provided between the central part (22) and the two end parts (18, 20).

6. Container according to Claim 5, **characterized in that** the seals (94) for bridging the annular gap (90) may be pressed against an opposite sealing face (44, 36) by way of an impingement by a pressurized fluid, and may be retracted from the sealing face (44, 36) by applying a vacuum.

7. Container according to Claim 5 or 6, **characterized in that** the seals (94) are inserted into grooves (92) of the end parts (18, 20) and may be pressed against opposite sealing faces (44, 36) of the central part (22).

8. Container according to one of the preceding claims, **characterized in that** the central part (22), in the vicinity of at least one of the two end parts (18, 20), is provided with a screen or a perforated plate (54) that is permeable to the treatment medium and that retains the items in the central part (22).

9. Container according to one of the preceding claims, **characterized in that** one of the two end parts (20), in the vicinity of the central part (22), is provided with a screen or a perforated plate (54) that is permeable to the treatment medium and that retains the items in the central part (22).

10. Container according to one of the preceding claims, **characterized in that** the central part (22) is subdivided into individual compartments (52) which each receive part of the items.

11. Container according to one of Claims 3 to 9, **characterized in that** the end parts (18, 20), along part of the circumference thereof, are provided with a collar (98) which projects beyond the connection plane (26, 28) and which serves for centring the central part (22) when said parts introduced into the intermediate space (24).

12. Container according to Claim 11, **characterized in that** the collar (98) engages about a projecting periphery (99) of the central part (22) that is introduced into the intermediate space (24).

13. Method for treating comparatively small items such as parts of syringes, closures, or closure caps of rubber, plastics, or aluminium, for closing containers for pharmaceutical purposes or the like, wherein a container (12) which in an upright position has a tub-shaped lower end part (18), an upwardly tapered upper end part (20), and a central part (22), disposed between the end parts, is loaded with the items, wherein the items in the central part (22) of the container (12) are impinged upon by a treatment medium which is supplied through a connector (88) at the upper end of the upper end part (18), or through a connector (48) at the deepest point of the lower end part (20), to one of the end parts (18; 20), from there is directed through the central part (22), and is discharged from the other of the end parts (20; 18), **characterized in that** the central part (22) which is inserted as a separate component into an intermediate space (24) between the two end parts (20, 18) is removed from the intermediate space (24) for loading and unloading of the container (12).

14. Method according to Claim 13, **characterized in that** the comparatively small items are contained in bags or sachets which, for loading and unloading the container (12), are placed from above into the central part (22), or are upwardly removed from the central part (22), respectively, after the latter has been removed from in between the two end parts (18, 20).

## Revendications

1. Récipient (12) pour le traitement de petits objets, tels que des pièces de seringues, des fermetures ou des capuchons de fermeture en caoutchouc, en plastique ou en aluminium, pour fermer des récipients à usage pharmaceutique ou similaire, le récipient (12) présentant, en position debout, une partie d'extrémité inférieure en forme de cuve (18) et une partie d'extrémité supérieure rétrécie vers le haut (20), la partie d'extrémité inférieure (18) étant pourvue, au niveau de sa partie la plus profonde, et la partie d'extrémité supérieure (20) étant pourvue, au niveau de l'extrémité supérieure, de raccords (48, 88) pour l'alimentation et l'évacuation d'au moins un milieu de traitement, et le récipient (12) présentant une partie centrale (22) disposée entre les parties d'extrémité pour recevoir les objets à nettoyer, à travers laquelle peut être guidé le milieu de traitement acheminé dans l'une des parties d'extrémité (18 ; 20), pour être évacué à travers l'autre partie d'extrémité (20 ; 18), **caractérisé en ce que** la partie centrale (22) est insérée en tant que composant séparé dans un espace intermédiaire (24) entre les deux parties d'extrémité (18, 20) et peut être enlevée au besoin hors de l'espace intermédiaire (24).

2. Récipient selon la revendication 1, **caractérisé en ce que** la partie centrale (22) peut être connectée de manière étanche aux gaz et aux liquides aux deux parties d'extrémité (18, 20).

3. Récipient selon la revendication 1 ou 2, **caractérisé en ce que** la partie centrale (22) peut être connectée aux deux parties d'extrémité (18, 20) dans deux plans de connexion parallèles (26, 28) et peut être enlevée de l'espace intermédiaire (24) dans une direction parallèle aux plans de connexion (26, 28).

4. Récipient selon l'une quelconque des revendications précédentes, **caractérisé en ce que** lors de l'enlèvement et de l'introduction de la partie centrale (22), les deux parties d'extrémité (18, 20) restent stationnaires et les dimensions de l'espace intermédiaire (24) entre les deux parties d'extrémité (18, 20) restent inchangées.

5. Récipient selon l'une quelconque des revendications précédentes, **caractérisé en ce qu'**entre la partie centrale 22) et les deux parties d'extrémité (18, 20) sont prévus des joints d'étanchéité annulaires (94) qui surmontent les fentes annulaires (90) entre les parties adjacentes (18, 22 ; 20, 22).

6. Récipient selon la revendication 5, **caractérisé en ce que** les joints d'étanchéité (94), pour surmonter la fente annulaire (90), pressent par sollicitation avec un fluide sous pression contre une surface d'étanchéité opposée (44, 36) et par application d'une dépression, peuvent être retirés de la surface d'étanchéité (44, 36).

7. Récipient selon la revendication 5 ou 6, **caractérisé en ce que** les joints d'étanchéité (94) sont insérés dans des rainures (92) des parties d'extrémité (18, 20) et peuvent être pressés contre des surfaces d'étanchéité opposées (44, 36) de la partie centrale (22).

8. Récipient selon l'une quelconque des revendications précédentes, **caractérisé en ce que** la partie centrale (22) est pourvue à proximité d'au moins l'une des deux parties d'extrémité (18, 20) d'un tamis ou d'une tôle perforée (54) perméable au milieu de traitement, qui retient les objets dans la partie centrale (22).

9. Récipient selon l'une quelconque des revendications précédentes, **caractérisé en ce que** l'une des deux parties d'extrémité (20) est pourvue à proximité de la partie centrale (22) d'un tamis ou d'une tôle perforée (54) perméable au milieu de traitement, qui retient les objets dans la partie centrale (22).

10. Récipient selon l'une quelconque des revendications précédentes, **caractérisé en ce que** la partie centrale (22) est divisée en compartiments individuels (52) qui reçoivent à chaque fois une partie des objets.

11. Récipient selon l'une quelconque des revendications 3 à 9, **caractérisé en ce que** les parties d'extrémité (18, 20) sont pourvues le long d'une partie de leur périphérie d'un collet (98) faisant saillie au-delà du plan de connexion (26, 28), qui sert au centrage de la partie centrale (22) lors de l'introduction dans l'espace intermédiaire (24).

12. Récipient selon la revendication 11, **caractérisé en ce que** le collet (98) vient en prise autour d'un bord saillant (99) de la partie centrale (22) introduite dans l'espace intermédiaire (24).

13. Procédé pour le traitement de petits objets, tels que des pièces de seringues, des fermetures ou des capuchons de fermeture en caoutchouc, en plastique ou en aluminium, pour fermer des récipients à usage pharmaceutique ou similaire, un récipient (12), qui, en position debout, présente une partie d'extrémité inférieure en forme de cuve (18), une partie d'extrémité supérieure rétrécie vers le haut (20) et une partie centrale (22) disposée entre elles, étant chargé avec les objets, les objets étant sollicités dans la partie centrale (22) du récipient (12) avec un milieu de traitement qui est acheminé à travers un raccord (88) à l'extrémité supérieure de la partie d'extrémité supérieure (18) ou à travers un raccord (48) au niveau de la partie la plus profonde de la partie d'extrémité inférieure (20) dans l'une des parties d'extrémité (18 ;20), est guidé de là à travers la partie centrale (22) et est évacué hors de l'autre des parties d'extrémité (20 ; 18), **caractérisé en ce que** la partie centrale (22) insérée en tant que composant séparé dans un espace intermédiaire (24) entre les deux parties d'extrémité (18, 20) est enlevée de l'espace intermédiaire (24) pour le chargement et le déchargement du récipient (12).

14. Procédé selon la revendication 13, **caractérisé en ce que** les petits objets sont contenus dans des sacs ou des sachets qui, pour le chargement et le déchargement du récipient (12) sont placés par le haut dans la partie centrale (22) ou sont enlevés vers le haut hors de la partie centrale (22), après que celle-ci a été enlevée d'entre les deux parties d'extrémité (18, 20).
